# DEMANDE DE BREVET EUROPEEN

(11) **EP 1 607 393 A1**
(43) Date de publication de la demande: **21.12.2005**
(21) Numéro de dépôt: 05291308.4
(22) Date de dépôt: 17.06.2005
(51) Int. Cl.: C07D 311/82, A61K 7/13, C07D 241/46, C07D 265/38, C07D 279/22

(54) **Compositions comprenant des colorants directs hydroxyalkyles, procédés de mise en oeuvre et utilisations.**

(30) Priorité: 18.06.2004 FR 0406675
(71) Demandeur: L'OREAL, 75008 Paris (FR)
(72) Inventeur: Lagrange, Alain, 77700 Coupvray (FR)
(74) Mandataire: Dossmann, Gérard

(57) **Abrégé**

La présente invention concerne également une composition cosmétiquee pour la coloration des fibres kératiniques, en particulier des fibres kératiniques humaines telles que les cheveux comprenant dans un milieu de coloration approprié au moins un composé hydroxyalkylé de formule générale (I) suivante 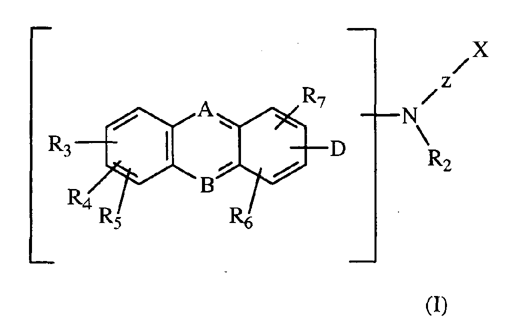 et au moins un adjuvant cosmétiqueselon l'invention.

La présente invention concerne aussi un procédé de coloration des fibres kératiniques mettant en oeuvre ces compositions à base de composés hydroxyalkylés, un dispositif à compartiments et l'utilisation de ces composés en coloration capillaire.

## Description

La présente demande a pour objet des compositions cosmétiques comprenant des composés hydroxyalkylés pour la coloration directe des fibres kératiniques, en particulier des fibres kéràtiniques humaines, telles que les cheveux, des procédés mettant en oeuvre cette composition, un dispositif à compartiments et l'utilisation des composés hydroxyalkylés.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho-ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques par une coloration directe ou semi-permanente. Le procédé classiquement utilisé en coloration directe consiste à appliquer sur les fibres kératiniques des colorants directs qui sont des molécules colorées et colorantes ayant une affinité pour les fibres, à laisser pauser pour permettre aux molécules colorées de pénétrer par diffusion à l'intérieur du cheveu, puis à rincer les fibres.

Il est connu par exemple d'utiliser des colorants directs nitrés benzèniques, anthraquinoniques, nitropyridiniques, azoïques, xanthéniques, acridiniques, aziniques ou triarylméthaniques.

Il en résulte des colorations qui peuvent être particulièrement chromatiques qui sont cependant temporaires ou semi-permanentes à cause de la nature des liaisons entre les colorants directs et la fibre kératinique. Ces interactions font que la désorption des colorants de la surface et/ou du coeur de la fibre se fait facilement. Les colorations présentent généralement une faible puissance tinctoriale et une mauvaise tenue aux lavages ou à la transpiration.

Contrairement aux compositions de coloration par oxydation, les compositions de coloration directe ou semi-permanente sont généralement mises en oeuvre sans la présence d'un agent oxydant. Ces colorations peuvent être effectuées de manière répétée sans dégrader la fibre kératinique.

Il existe un réel besoin de disposer de compositions de coloration directe améliorées en termes d'innocuité, de ténacité et de sélectivité.

De manière surprenante et avantageuse, la Demanderesse vient de découvrir une nouvelle famille de composés hydroxyalkylés utilisés en tant que colorants directs pour la préparation de compositions de coloration des fibres kératiniques, en particulier des fibres kératiniques humaines telles que les cheveux, permettant ces améliorations.

Outre leur avantage en terme d'innocuité, les colorants directs hydroxyalkylés selon la présente demande aboutissent à des colorations résistantes aux agents extérieurs (soleil, intempéries) ainsi qu'aux shampooings et à la transpiration.

En outre, les compositions comprenant ces colorants directs hydroxyalkylés présentent un bon profil toxicologique.

Un premier objet de la présente invention consiste en une composition cosmétique pour la coloration des fibres kératiniques, en particulier des fibres kératiniques humaines telles que les cheveux comprenant dans un milieu de coloration approprié au moins un colorant direct hydroxyalkylé particulier et au moins un adjuvant cosmétique.

Un second objet de la présente demande consiste en un procédé de coloration des fibres kératiniques, en particulier des fibres kératiniques humaines telles que les cheveux mettant en oeuvre ces compositions à base de colorants directs hydroxyalkylés.

Un autre objet de la présente demande porte sur un dispositif à compartiments comprenant la composition selon l'invention.

Un autre objet de la présente demande consiste en l'utilisation de colorants directs hydroxyalkylés selon la présente invention pour la coloration des fibres kératiniques, en particulier les fibres kératiniques humaines telles que les cheveux.

D'autres caractéristiques, aspects, objets et avantages de la présente invention apparaîtront encore plus clairement à la lecture de la description et des exemples qui suivent.

L'invention porte sur une composition cosmétique pour la coloration des fibres kératiniques, en particulier les fibres kératiniques humaines telles que les cheveux comprenant dans un milieu de coloration approprié :
- au moins un composé hydroxyalkylé de formule (I) suivante : 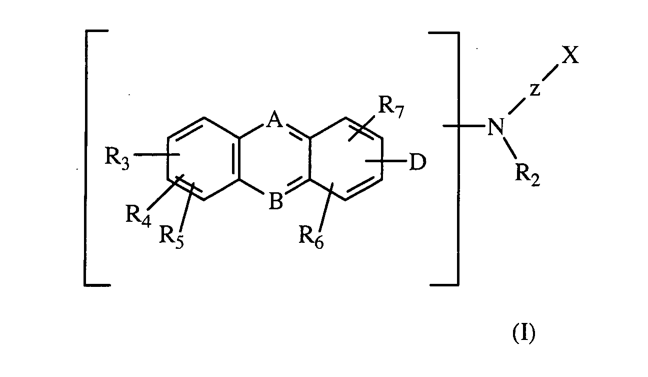 dans laquelle :
   A représente un atome d'azote, un groupe NR₉ ou CR₉,
   B représente un atome d'oxygène, de soufre ou d'azote ou un groupement -CR₉ ;
   A et B représentant au moins un hétéroatome portant une charge cationique permanente,
   l'électroneutralité du composé de formule (I) est assurée par un contre ion Y externe et/ou par un des substituants porté par le noyau tricyclique ;
   D représente un groupe amino non substitué, mono- ou disubstitué par un ou plusieurs groupements alkyle en C₁-C₂₄, linéaire ou ramifié, pouvant être interrompu par un ou plusieurs hétéroatomes et/ou pouvant être substitué, le ou les substituants du groupe amino pouvant former ensemble un hétérocycle saturé ou insaturé, éventuellement aromatique comprenant 5 à 12 chaînons, ces substituants pouvant eux-mêmes être substitués par un acide sulfonique et/ou un acide carboxylique ; un groupe amino mono- ou disubstitué par un groupement aryle éventuellement substitué ;
   D se situant en position para du carbone qui porte A ou en position para du carbone qui porte B ;
   le substituant -N(zX)R₂ est porté par le noyau tricyclique ;
   z représente un radical alkylène en C₁-C₂₄, linéaire ou ramifié, éventuellement interrompu par un ou plusieurs hétéroatomes, de préférence l'oxygène, portant un substituant X de formule suivante (II) : 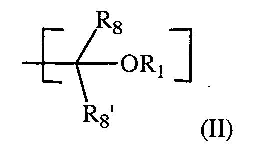
   R₁ représente un atome d'hydrogène ou un groupe hydroxyalkyle en C₁-C₂₄;
   R₈ et R₈' représentent, indépendamment l'un de l'autre, un atome d'hydrogène ou un groupe alkyle en C₁-C₂₄ pouvant être interrompu par un ou plusieurs hétéroatomes ou un groupement carbonyle et/ou pouvant être substitué ;
   R₂ représente un atome d'hydrogène ; un radical alkyle en C₁-C₂₄, linéaire ou ramifié, pouvant être interrompu par un ou plusieurs hétéroatomes ou un groupement carbonyle et/ou pouvant être substitué ; ou un groupe hydroxyalkyle ;
   R₃, R₄, R₅, R₆ et R₇ représentent, indépendamment les uns des autres, un atome d'hydrogène ou d'halogène ; un radical alkyle en C₁-C₂₄, linéaire ou ramifié, pouvant être interrompu par un ou plusieurs hétéroatomes ou un groupement carbonyle et/ou pouvant être substitué ; un groupe amino non substitué, mono- ou disubstitué par un ou plusieurs groupements alkyle en C₁-C₂₄, linéaire ou ramifié, pouvant être interrompus par un ou plusieurs hétéroatomes ou groupements carbonyle et/ou pouvant être substitués, le ou les substituants du groupe amino pouvant former ensemble un hétérocycle saturé ou non, éventuellement aromatique comprenant 5 à 12 chaînons ; éventuellement substitué par un acide carboxylique et/ou un acide sulfonique ;
   lorsque R₂ représente un atome d'hydrogène, alors D, R₃, R₄, R₅, R₆ ou R₇ représente un groupe amino disubstitué ;
   R₉ représente un groupement alkyle en C₁-C₂₄, linéaire ou ramifié, substitué ou non substitué, un radical benzyle ou aryle éventuellement substitué par au moins un groupe alkyle en C₁-C₂₄, au moins un groupe alcoxy en C₁-C₂₄, au moins un groupement acide carboxylique et/ou au moins un acide sulfonique ;
   et leurs formes mésomères ; et
   - au moins un adjuvant cosmétique.

De préférence, la composition comprend le composé hydroxyalkylé de formule (III) : 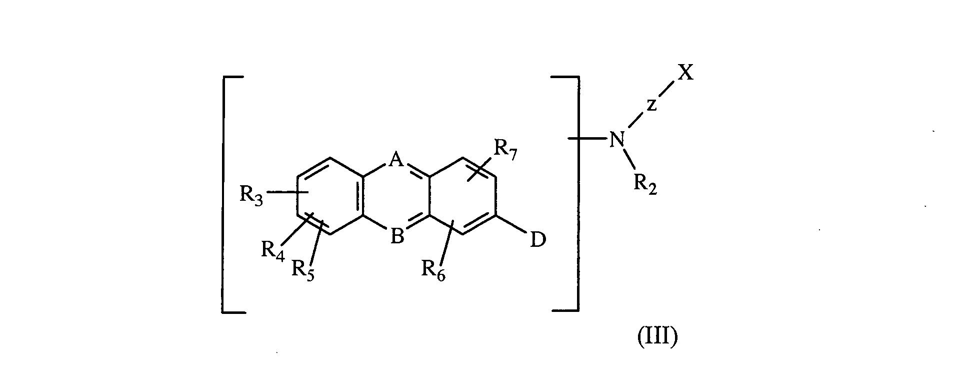 dans laquelle :
A, B, D, -N(zX)R₂, R₁, R₈ et R₈', R₂, R₃, R₄, R₅, R₆, R₇, R₉ ont la même signification que dans le composé de formule (I) défini précédemment.

De préférence, la composition comprend le composé de formule (V) : 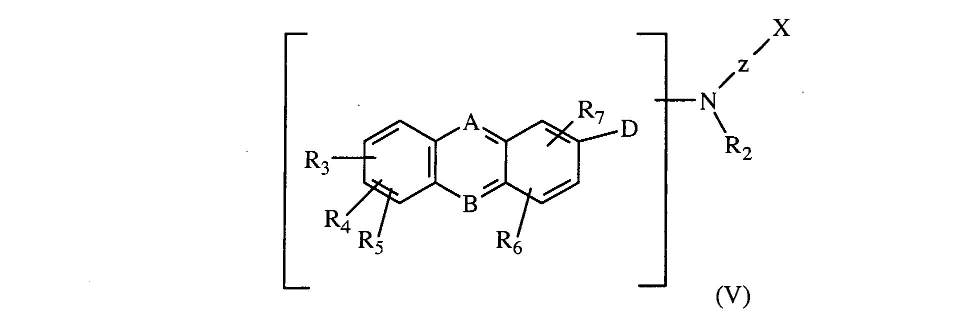 dans laquelle :
A, B, D, -N(zX)R₂, R₁, R₈ et R₈', R₂, R₃, R₄, R₅, R₆, R₇, R₉ ont la même signification que dans le composé de formule (I) défini précédemment.

Par le terme « substitué », on entend substitué par un groupement alkyle en C₁-C₆ linéaire ou ramifié ; un groupement hydroxyalkyle en C₁-C₆ linéaire ou ramifié ; un groupement acétylamino ; un groupement hydroxyle ; un groupement cyano ; un atome d'halogène, de préférence le fluor, le brome ; un groupement alcoxy en C₁-C₆ linéaire ou ramifié ; un groupement alkylcarbonyle en C₁-C₆ linéaire ou ramifié ; un groupement hydroxycarbonyle ; un groupement alcoxycarbonyle en C₁-C₆ linéaire ou ramifié ; un groupement aryle substitué par un des substituants précités .

De préférence, R₃, R₄, R₅, R₆ et R₇ représentent un atome d'hydrogène.

Selon un mode de réalisation plus particulier, les groupements D et -N(zX)R₂, sont situés sur des cycles aromatiques différents.

Conformément à une variante préférée, le groupement -N(zX)R₂ se situe en position para du carbone qui porte A ou en position para du carbone qui porte B.

De manière préférée, les composés de formule (I) selon la présente invention sont :

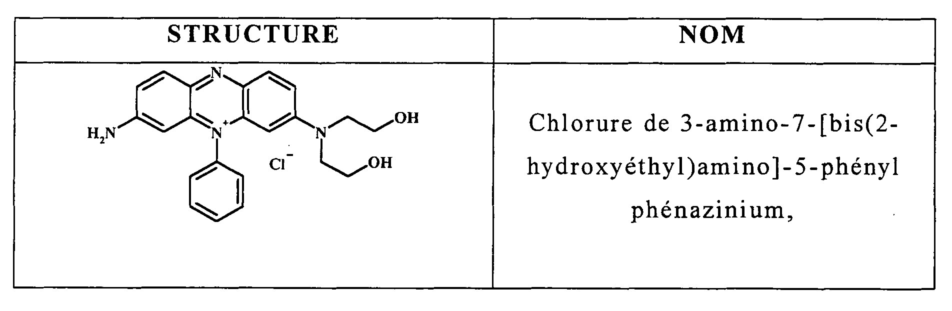

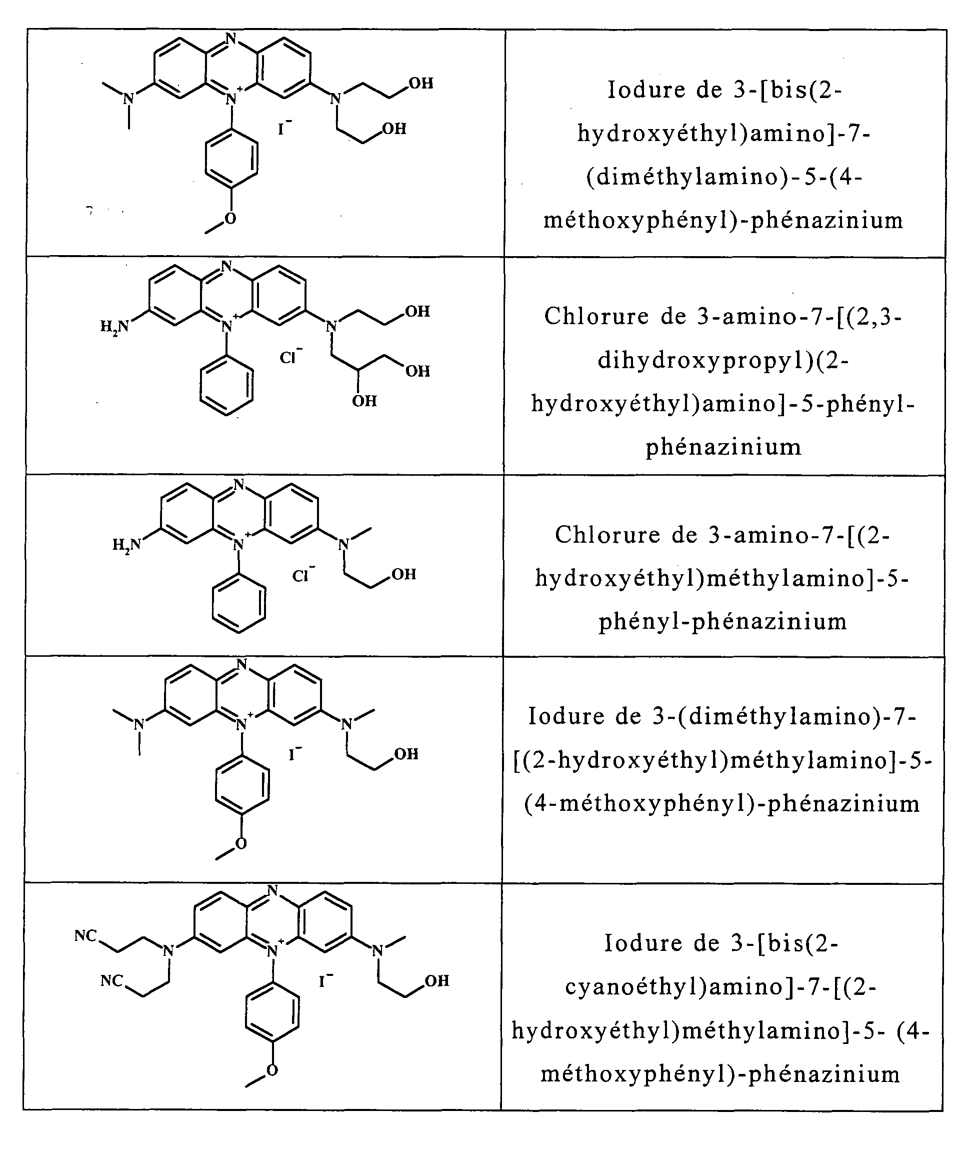

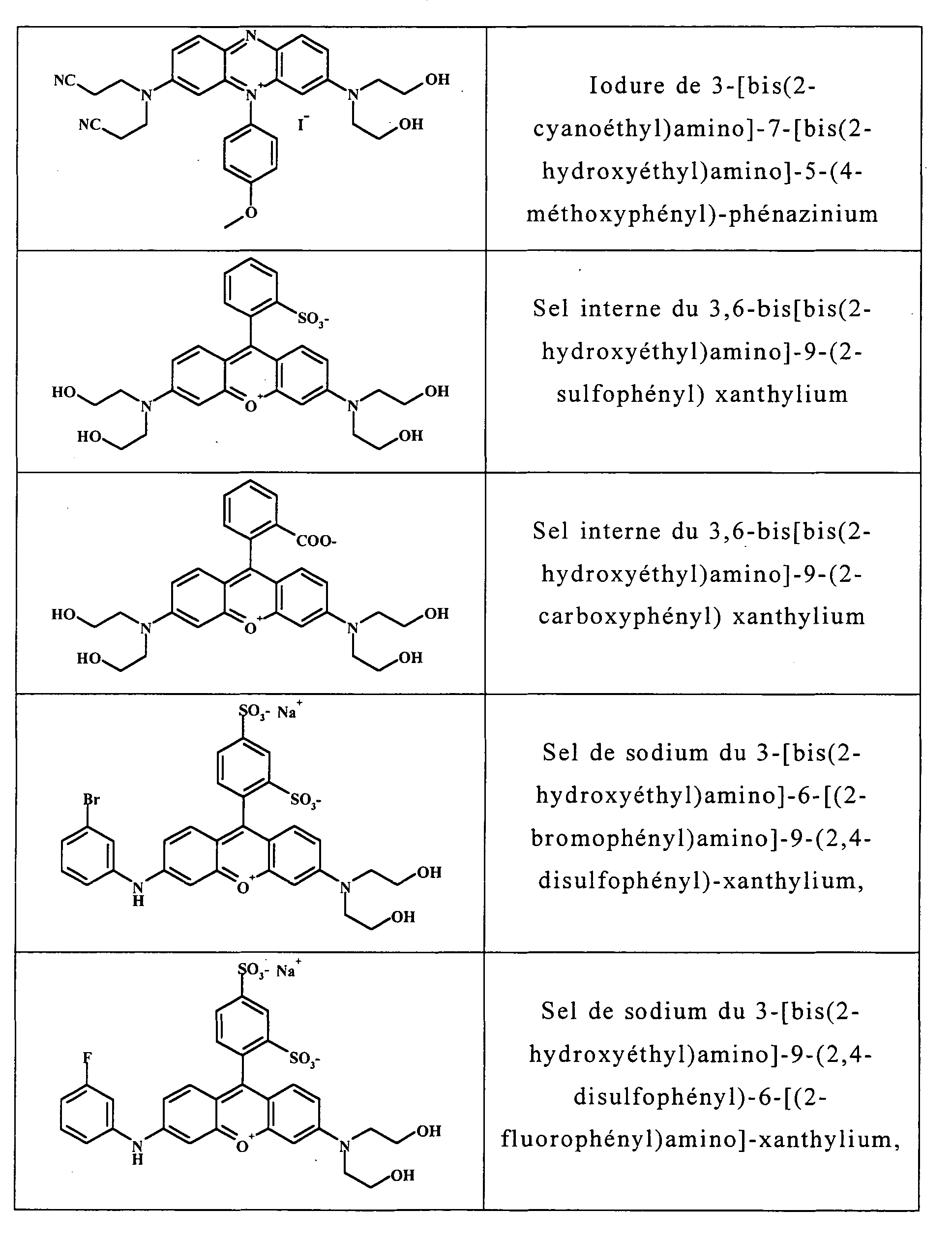

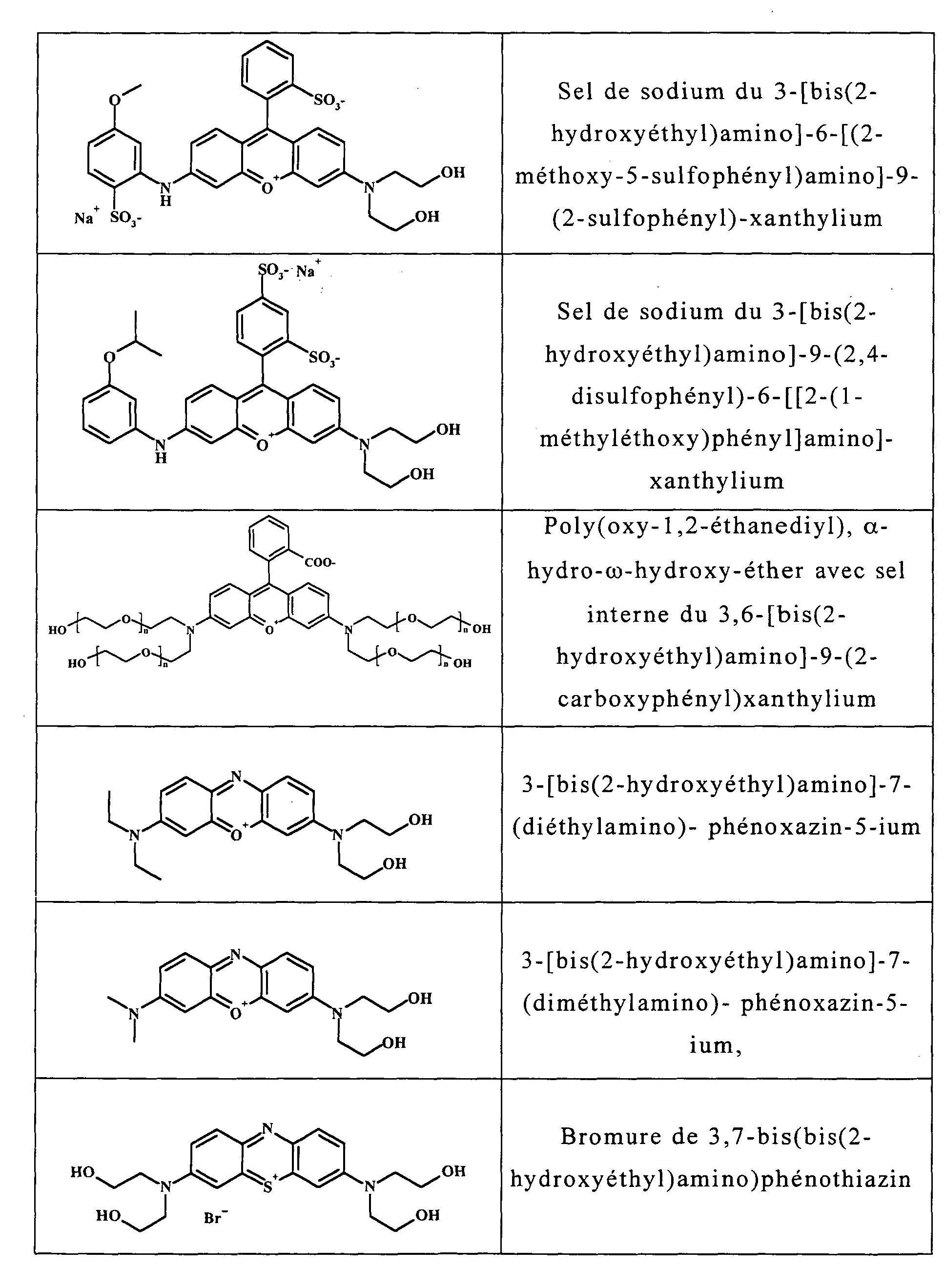

Par mésomères, on entend un composé dans lequel les doubles liaisons peuvent se délocaliser par résonance. Des exemples de formes mésomères de certains des composés préférés sont exposés ci-dessous.

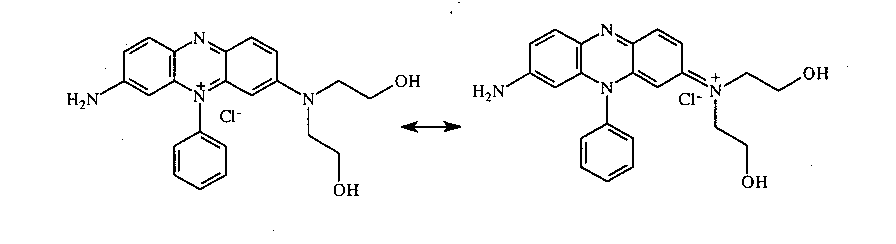

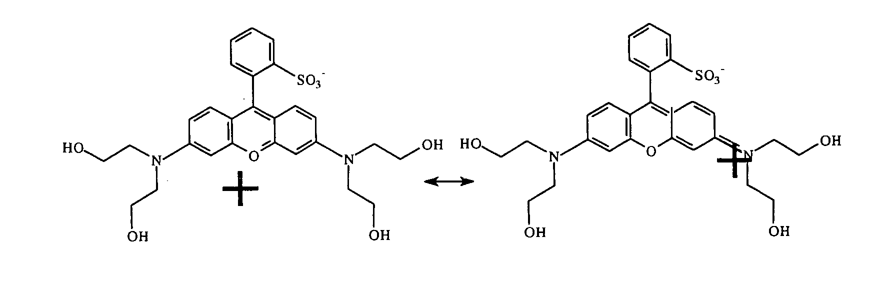

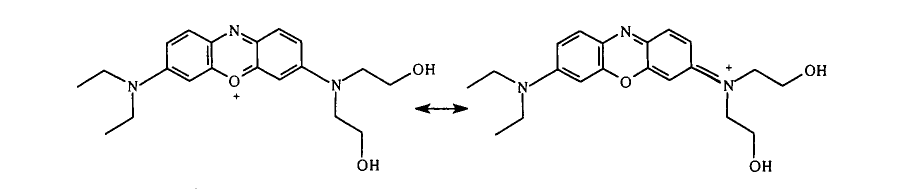

Le composé de formule (I) porte au moins une charge cationique permanente sur un hétéroatome du groupe A ou B. Cette charge est due à un nombre de liaison de coordination supérieur au nombre de valence de l'hétéroatome. Elle est de ce fait indépendante du pH du milieu.

Cette charge cationique peut être neutralisée par un contre-ion se trouvant au sein même du composé comme par exemple un sulfonate ou un carboxylate porté par les substituants du noyau tricyclique ou par R₉.

Lorsque cette charge cationique n'est pas neutralisée par un contre-ion interne, l'électroneutralité du composé de formule (I) est assurée par un contre-ion Y externe audit composé.

Y est un contre-ion anionique choisi parmi un iodure, un bromure, un chlorure, un éthylsulfate, un méthylsulfate.

Lorsque le composé de formule (I) porte un nombre de charge négative supérieur au nombre de charge cationique, alors le contre-ion Y est cationique.

Y est un contre-ion cationique choisi parmi un sodium, un potassium.

De préférence, le composé de formule (I) porte une seule charge cationique permanente sur un hétéroatome du groupe A ou B.

La composition à base de ces colorants directs hydroxyalkylés de formule (I) selon la présente invention comprend de 0,001 à 20%, de préférence de 0,01 à 10 %, de préférence de 0,1 à 5 % en poids de colorant(s) direct(s) de formule (I) par rapport au poids total de la composition.

Le ou les adjuvants cosmétiques contenus dans la composition selon l'invention sont choisis parmi les agents tensio-actifs, les polymères, les céramides et pseudo-céramides, les vitamines et pro-vitamines, les filtres solaires, les composés solides tels que les pigments, les agents nacrants ou opacifiants, les colorants directs autres que ceux de formule (I), les précurseurs de colorant d'oxydation, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les solvants, les agents de pénétration, les tampons, les agents dispersants, les agents de conditionnement, et les agents conservateurs

Les adjuvants ci-dessus sont en général présents en quantité comprise pour chacun d'eux entre 0,01 et 20 % en poids par rapport au poids total de la composition.

Bien entendu, l'homme de l'art veillera à choisir ce ou ces composés de manière telle que les propriétés avantageuses attachées intrinsèquement à la composition de coloration directe conforme à l'invention ne soient pas, ou substantiellement pas, altérées par la ou les adjonctions envisagées.

Le ou les colorants directs additionnels selon l'invention sont choisis parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention, on peut citer de manière non limitative les composés suivants :
- 1,4-diamino-2-nitrobenzène,
- 1-amino-2 nitro-4-β- hydroxyéthylaminobenzène
- 1-amino-2 nitro-4-bis(β-hydroxyéthyl)-aminobenzène
- 1,4-Bis(β -hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1- β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoïques cationiques décrits dans les demandes de brevets WO 95/15144, WO-95/01772 et EP-714954 dont le contenu fait partie intégrante de l'invention.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1, 4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs additionnels représentent de préférence de 0,001 à 20% en poids environ du poids total de la composition prête à l'emploi et encore plus préférentiellement de 0,001 à 10% en poids environ.

Lorsque l'adjuvant cosmétique est un précurseur de colorant d'oxydation, celui-ci est choisi parmi une ou plusieurs bases d'oxydation et/ou un ou plusieurs coupleurs.

A titre d'exemple, les bases d'oxydation sont choisies parmi les phénylènediamines , les bis-phénylalkylènediamines, les para-aminophénols, les ortho-aminophénols, les bases hétérocycliques autres que les paraphénylènediamines hétérocycliques de formule (I) et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi citer le 4,5-diamino-1-(β-méthoxyéthyl)pyrazole.

La ou les bases d'oxydation additionnelles présentes dans la composition de l'invention sont en général présentes en quantité comprise allant de 0,001 à 20 % en poids environ du poids total de la composition tinctoriale, de préférence allant de 0,005 à 6 %.

Si la composition selon l'invention contient au moins une base d'oxydation, elle contient de préférence un ou plusieurs coupleurs conventionnellement utilisés pour la coloration de fibres kératiniques. Parmi ces coupleurs, on peut notamment citer les métaphénylènediamines, les métaaminophénols, les métadiphénols, les coupleurs naphtaléniques, les coupleurs hétérocycliques ainsi que leur sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en quantité comprise allant de 0,001 à 20 % en poids environ du poids total de la composition tinctoriale, de préférence allant de 0,01 à 10 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Les agents oxydants classiquement utilisés pour la coloration d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

Le milieu approprié pour la coloration appelé aussi support de coloration est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le glycérol, les mono éthers de polyols, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, de préférence entre 5 et 11 environ, et encore plus particulièrement de 6 à 8,5. Il peut être ajusté à la valeur désirée au moyen d'agents acidifiants ou alcalinisants habituellement utilisés en coloration des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 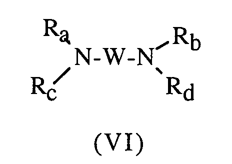 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une coloration des fibres kératiniques, et notamment des cheveux humains.

Le procédé de la présente invention est un procédé dans lequel on applique sur les fibres la composition selon la présente invention telle que définie précédemment, on laisse pauser la composition pendant une période comprise 5 minutes et 1 heure, de préférence entre 15 minutes et 1 heure, puis on rince lesdites fibres.

Selon un mode de réalisation particulier, la composition de l'invention exempte d'agent oxydant est appliquée sur les fibres kératiniques en présence d'un agent oxydant pendant un temps suffisant pour obtenir l'éclaircissement souhaité. L'agent oxydant peut être ajouté à la composition de l'invention juste au moment de l'emploi, ou il peut être mis en oeuvre à partir d'une composition oxydante le contenant, appliquée simultanément ou séquentiellement à la composition de l'invention.

Selon un mode de réalisation particulier, la composition selon la présente invention exempte d'agent oxydant comprend au moins un précurseur de colorant d'oxydation, et elle est mélangée, de préférence au moment de l'emploi, à une composition contenant, dans un milieu approprié pour la coloration, au moins un agent oxydant. Le mélange obtenu est ensuite appliqué sur les fibres kératiniques. Après un temps de pose de 5 minutes à 1 heure environ, de préférence 15 minutes à 1 heure environ, les fibres kératiniques sont rincées, lavées au shampooing, rincées à nouveau puis séchées.

La composition oxydante peut également renfermer divers adjuvants utilisés classiquement dans les compositions pour la coloration des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, encore plus préférentiellement entre 5 et 11 et encore plus particulièrement entre 6 et 8,5. Il peut être ajusté à la valeur désirée au moyen d'agents acidifiants ou alcalinisants habituellement utilisés en coloration des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une coloration des fibres kératiniques, et notamment des cheveux humains.

L'invention a aussi pour objet un dispositif à plusieurs compartiments ou "kit" de coloration dans lequel un premier compartiment renferme la composition tinctoriale selon l'invention exempte d'agent oxydant et un deuxième compartiment renferme un agent oxydant. Le compartiment contenant au moins un colorant de formule (I) peut éventuellement contenir au moins un précurseur de colorant d'oxydation défini ci-dessus. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLES

### Exemple 1

La composition selon l'invention a été préparé:

| Composants | quantité |
|---|---|
| Bromure de 3,7-bis(bis(2-hydroxyéthyl)amino)phénothiazin-5-ium | 0,1 g |
| Ethanol | 5,0 g |
| Alkylpolyglucoside | 4,0 g MA |
| Conservateur | qs |
| 2-amino-2-méthyl-1-propanol | qs pH 7,5 |
| Eau | qsp 100 |

Cette composition permet d'obtenir des nuances bleues intenses sur les cheveux.

### Exemple 2

La composition selon l'invention a été préparé:

| Composants | quantité |
|---|---|
| sel interne du 3,6-bis[bis(2-hydroxyéthyl)amino]-9-(2-carboxyphényl) xanthylium | 0,1 g |
| Ethanol | 5,0 g |
| Alkylpolyglucoside | 4,0 g MA |
| Conservateur | qs |
| 2-amino-2-méthyl-1-propanol | qs pH 7,5 |
| Eau | qsp 100 |

La composition permet d'obtenir des nuances rose-fuchsia brillante sur les cheveux.

## Revendications

1. Composition cosmétique pour la coloration des fibres kératiniques, en particulier des fibres kératiniques humaines telles que les cheveux comprenant dans un milieu de coloration approprié :
- au moins un composé hydroxyalkylé de formule (I) suivante : 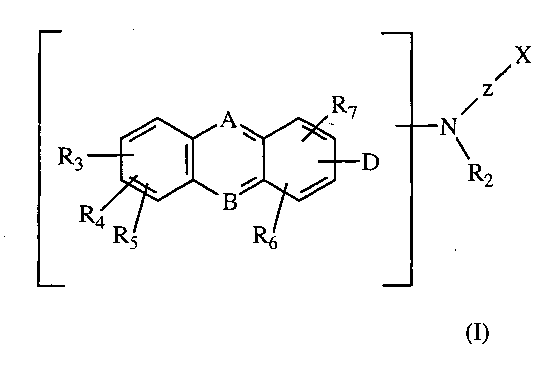 dans laquelle :
A représente un atome d'azote, un groupe NR₉ ou CR₉,
B représente un atome d'oxygène, de soufre ou d'azote ou un groupement -CR₉ ;
A et B représentant au moins un hétéroatome portant une charge cationique permanente,
l'électroneutralité du composé de formule (I) est assurée par un contre ion Y externe et/ou par un des substituants porté par le noyau tricyclique ;
D représente un groupe amino non substitué, mono- ou disubstitué par un ou plusieurs groupements alkyle en C₁-C₂₄, linéaire ou ramifié, pouvant être interrompu par un ou plusieurs hétéroatomes et/ou pouvant être substitué, le ou les substituants du groupe amino pouvant former ensemble un hétérocycle saturé ou insaturé, éventuellement aromatique comprenant 5 à 12 chaînons, ces substituants pouvant eux-mêmes être substitués par un acide sulfonique et/ou un acide carboxylique ; un groupe amino mono- ou disubstitué par un groupement aryle éventuellement substitué ;
D se situant en position para du carbone qui porte A ou en position para du carbone qui porte B ;
le substituant -N(zX)R₂ est porté par le noyau tricyclique ;
z représente un radical alkylène en C₁-C₂₄, linéaire ou ramifié, éventuellement interrompu par un ou plusieurs hétéroatomes, de préférence l'oxygène, portant un substituant X de formule suivante (II) : 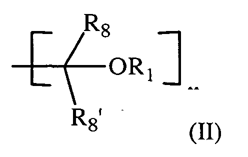
R₁ représente un atome d'hydrogène ou un groupe hydroxyalkyle en C₁-C₂₄;
R₈ et R₈' représentent, indépendamment l'un de l'autre, un atome d'hydrogène ou un groupe alkyle en C₁-C₂₄ pouvant être interrompu par un ou plusieurs hétéroatomes ou un groupement carbonyle et/ou pouvant être substitué ;
R₂ représente un atome d'hydrogène ; un radical alkyle en C₁-C₂₄, linéaire ou ramifié, pouvant être interrompu par un ou plusieurs hétéroatomes ou un groupement carbonyle et/ou pouvant être substitué ; ou un groupe hydroxyalkyle ;
R₃, R₄, R₅, R₆ et R₇ représentent, indépendamment les uns des autres, un atome d'hydrogène ou d'halogène ; un radical alkyle en C₁-C₂₄, linéaire ou ramifié, pouvant être interrompu par un ou plusieurs hétéroatomes ou un groupement carbonyle et/ou pouvant être substitué ; un groupe amino non substitué, mono- ou disubstitué par un ou plusieurs groupements alkyle en C₁-C₂₄, linéaire ou ramifié, pouvant être interrompus par un ou plusieurs hétéroatomes ou groupements carbonyles et/ou pouvant être substitué, le ou les substituants du groupe amino pouvant former ensemble un hétérocycle saturé ou non éventuellement aromatique comprenant 5 à 12 chaînons ; éventuellement substitué par un acide carboxylique et/ou un acide sulfonique ;
lorsque R₂ représente un atome d'hydrogène, alors D, R₃, R₄, R₅, R₆ ou R₇ représente un groupe amino disubstitué ;
R₉ représente un groupement alkyle en C₁-C₂₄, linéaire ou ramifié, substitué ou non substitué, un radical benzyle ou aryle éventuellement substitué par au moins un groupe alkyle en C₁-C₂₄, au moins un groupe alcoxy en C₁-C₂₄, au moins un groupement acide carboxylique et/ou au moins un acide sulfonique ;
et leurs formes mésomères ; et
- au moins un adjuvant cosmétique.

2. Composition selon la revendication 1, **caractérisée en ce que** le composé hydroxyalkylé est de formule (III) : 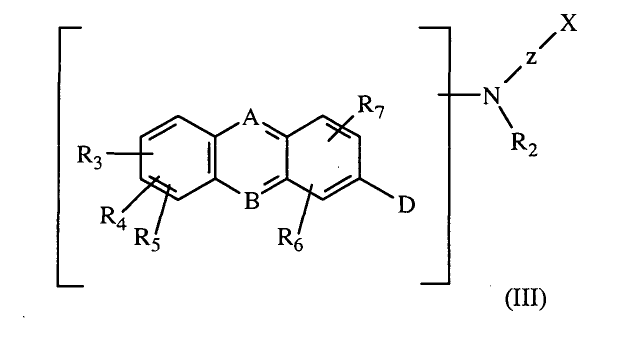 dans laquelle :
A représente un atome d'azote, un groupe NR₉ ou CR₉,
B représente un atome d'oxygène, de soufre ou d'azote ou un groupement -CR₉ ;
A et B représentant au moins un hétéroatome portant une charge cationique permanente,
l'électroneutralité du composé de formule (I) est assurée par un contre ion Y externe et/ou par un des substituants porté par le noyau tricyclique ;
D représente un groupe amino non substitué, mono- ou disubstitué par un ou plusieurs groupements alkyle en C₁-C₂₄, linéaire ou ramifié, pouvant être interrompu par un ou plusieurs hétéroatomes et/ou pouvant être substitué, le ou les substituants du groupe amino pouvant former ensemble un hétérocycle saturé ou insaturé, éventuellement aromatique comprenant 5 à 12 chaînons, ces substituants pouvant eux-mêmes être substitués par un acide sulfonique et/ou un acide carboxylique ; un groupe amino mono- ou disubstitué par un groupement aryle éventuellement substitué ;
le substituant -N(zX)R₂ est porté par le noyau tricyclique ;
z représente un radical alkylène en C₁-C₂₄, linéaire ou ramifié, éventuellement interrompu par un ou plusieurs hétéroatomes, de préférence l'oxygène, portant un substituant X de formule suivante (II) : 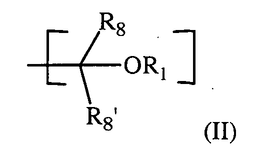
R₁ représente un atome d'hydrogène ou un groupe hydroxyalkyle en C₁-C₂₄;
R₈ et R₈' représentent, indépendamment l'un de l'autre, un atome d'hydrogène ou un groupe alkyle en C₁-C₂₄ pouvant être interrompu par un ou plusieurs hétéroatomes ou un groupement carbonyle et/ou pouvant être substitué ;
R₂ représente un atome d'hydrogène ; un radical alkyle en C₁-C₂₄, linéaire ou ramifié, pouvant être interrompu par un ou plusieurs hétéroatomes ou un groupement carbonyle et/ou pouvant être substitué ; ou un groupe hydroxyalkyle ;
R₃, R₄, R₅, R₆ et R₇ représentent, indépendamment les uns des autres, un atome d'hydrogène ou d'halogène ; un radical alkyle en C₁-C₂₄, linéaire ou ramifié, pouvant être interrompu par un ou plusieurs hétéroatomes ou un groupement carbonyle et/ou pouvant être substitué ; un groupe amino non substitué, mono- ou disubstitué par un ou plusieurs groupements alkyle en C₁-C₂₄, linéaire ou ramifié, pouvant être interrompus par un ou plusieurs hétéroatomes ou groupements carbonyles et/ou pouvant être substitué, le ou les substituants du groupe amino pouvant former ensemble un hétérocycle saturé ou non, éventuellement aromatique comprenant 5 à 12 chaînons ; éventuellement substitué par un acide carboxylique et/ou un acide sulfonique ;
lorsque R₂ représente un atome d'hydrogène, alors D, R₃, R₄, R₅, R₆ ou R₇ représente un groupe amino disubstitué ;
R₉ représente un groupement alkyle en C₁-C₂₄, linéaire ou ramifié, substitué ou non substitué, un radical benzyle ou aryle éventuellement substitué par au moins un groupe alkyle en C₁-C₂₄, au moins un groupe alcoxy en C₁-C₂₄, au moins un groupement acide carboxylique et/ou au moins un acide sulfonique ;
et leurs formes mésomères.

3. Composition selon la revendication 2, **caractérisée en ce que** le composé de formule (III) est choisi dans le groupe formé par :
- sel interne du 3,6-bis[bis(2-hydroxyéthyl)amino]-9-(2-sulfophényl) xanthylium ;
- sel de sodium du 3-[bis(2-hydroxyéthyl)amino]-6-[(2-bromophényl)amino]-9-(2,4-disulfophényl)-xanthylium ;
- sel de sodium du 3-[bis(2-hydroxyéthyl)amino]-9-(2,4-disulfophényl)-6-[(2-fluorophényl)amino]-xanthylium ;
- sel de sodium du 3-[bis(2-hydroxyéthyl)amino]-6-[(2-méthoxy-5-sulfophényl)amino]-9-(2-sulfophényl)-xanthylium ;
- sel de sodium du 3-[bis(2-hydroxyéthyl)amino]-9-(2,4-disulfophényl)-6-[[2-(1-méthyléthoxy)phényl]amino]-xanthylium ;
- poly(oxy-1,2-ethanediyl), α-hydro-ω-hydroxy- éther avec le sel interne du 3,6-[bis(2-hydroxyéthyl)amino]-9-(2-carboxyphenyl)xanthylium ;
- Sel interne du 3,6-bis[bis(2-hydroxyéthyl)amino]-9-(2-carboxyphényl) xanthylium,
- Bromure de 3,7-bis(bis(2-hydroxyéthyl)amino)phénothiazin-5-ium,
- 3-[bis(2-hydroxyéthyl)amino]-7-(diéthylamino)- phénoxazin-5-ium, et
- 3-[bis(2-hydroxyéthyl)amino]-7-(diméthylamino)- phénoxazin-5-ium.

4. Composition selon la revendication 1, **caractérisée en ce que** le composé est de formule (IV) : 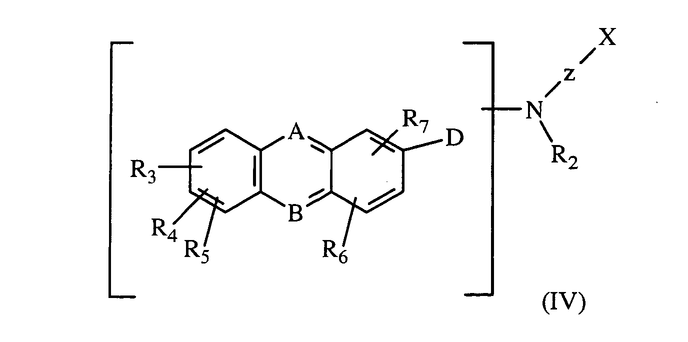 dans laquelle :
A représente un atome d'azote, un groupe NR₉ ou CR₉,
B représente un atome d'oxygène, de soufre ou d'azote ou un groupement -CR₉ ;
A et B représentant au moins un hétéroatome portant une charge cationique permanente,
l'électroneutralité du composé de formule (I) est assurée par un contre ion Y externe et/ou par un des substituants porté par le noyau tricyclique ;
D représente un groupe amino non substitué, mono- ou disubstitué par un ou plusieurs groupements alkyle en C₁-C₂₄, linéaire ou ramifié, pouvant être interrompu par un ou plusieurs hétéroatomes et/ou pouvant être substitué, le ou les substituants du groupe amino pouvant former ensemble un hétérocycle saturé ou insaturé, éventuellement aromatique comprenant 5 à 12 chaînons, ces substituants pouvant eux-mêmes être substitués par un acide sulfonique et/ou un acide carboxylique ; un groupe amino mono- ou disubstitué par groupement aryle éventuellement substitué ;
le substituant -N(zX)R₂ est porté par le noyau tricyclique ;
z représente un radical alkylène en C₁-C₂₄, linéaire ou ramifié, éventuellement interrompu par un ou plusieurs hétéroatomes, de préférence l'oxygène, portant un substituant X de formule suivante (II) : 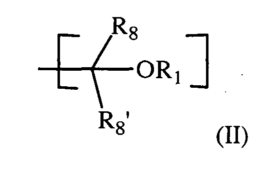
R₁ représente un atome d'hydrogène ou un groupe hydroxyalkyle en C₁-C₂₄;
R₈ et R₈' représentent, indépendamment l'un de l'autre, un atome d'hydrogène ou un groupe alkyle en C₁-C₂₄ pouvant être interrompu par un ou plusieurs hétéroatomes ou un groupement carbonyle et/ou pouvant être substitué ;
R₂ représente un atome d'hydrogène ; un radical alkyle en C₁-C₂₄, linéaire ou ramifié, pouvant être interrompu par un ou plusieurs hétéroatomes ou un groupement carbonyle et/ou pouvant être substitué ; ou un groupe hydroxyalkyle ;
R₃, R₄, R₅, R₆ et R₇ représentent, indépendamment les uns des autres, un atome d'hydrogène ou d'halogène ; un radical alkyle en C₁-C₂₄, linéaire ou ramifié, pouvant être interrompu par un ou plusieurs hétéroatomes ou un groupement carbonyle et/ou pouvant être substitué ; un groupe amino non substitué, mono- ou disubstitué par un ou plusieurs groupements alkyle en C₁-C₂₄, linéaire ou ramifié, pouvant être interrompus par un ou plusieurs hétéroatomes ou groupements carbonyles et/ou pouvant être substitué, le ou les substituants du groupe amino pouvant former ensemble un hétérocycle saturé ou non, éventuellement aromatique comprenant 5 à 12 chaînons ; éventuellement substitué par un acide carboxylique et/ou un acide sulfonique ;
lorsque R₂ représente un atome d'hydrogène, alors D, R₃, R₄, R₅, R₆ ou R₇ représente un groupe amino disubstitué ;
R₉ représente un groupement alkyle en C₁-C₂₄, linéaire ou ramifié, substitué ou non substitué, un radical benzyle ou aryle éventuellement substitué par au moins un groupe alkyle en C₁-C₂₄, au moins un groupe alcoxy en C₁-C₂₄, au moins un groupement acide carboxylique et/ou au moins un acide sulfonique ;
et leurs formes mésomères.

5. Composition selon la revendication 4, **caractérisée en ce que** le composé de formule (IV) est choisi parmi:
- chlorure de 3-amino-7-[bis(2-hydroxyéthyl)amino]-5-phényl phénazinium,
- iodure de 3-[bis(2-hydroxyéthyl)amino]-7-(diméthylamino)-5-(4-méthoxyphényl)-phénazinium ;
- chlorure de 3-amino-7-[(2-hydroxyéthyl)méthylamino]-5-phényl phénazinium,;
- chlorure de 3-amino-7-[(2,3-dihydroxypropyl)(2-hydroxyéthyl)amino]-5-phényl-phénazinium ;
- iodure de 3-(diméthylamino)-7-[(2-hydroxyéthyl)méthylamino]-5-(4-methoxyphényl)-phénazinium ;
- iodure de 3-[bis(2-cyanoéthyl)amino]-7-[(2-hydroxyéthyl)méthylamino]-5- (4-méthoxyphényl)-phénazinium ;
- iodure de 3-[bis(2-cyanoéethyl)amino]-7-[bis(2-hydroxyéthyl)amino]-5-(4-méthoxyphényl)-phénazinium ;

6. Composition cosmétique selon l'une quelconque des revendications précédentes, **caractérisée en ce que** le ou les composés hydroxyalkylés sont présents en une quantité allant de 0,001 à 20 %, de préférence de 0,01 à 10 % et de manière encore préférée de 0,1 à 5 % en poids par rapport au poids total de la composition.

7. Composition cosmétique selon l'une quelconque des revendications précédentes, **caractérisée en ce que** l'adjuvant cosmétique est choisi parmi les agents tensio-actifs, les polymères, les céramides et pseudo-céramides, les vitamines et pro-vitamines, les filtres solaires, les composés solides tels que les pigments, les agents nacrants ou opacifiants, les colorants directs autres que ceux de formule (I), les précurseurs de colorant d'oxydation, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les solvants, les agents de pénétration, les tampons, les agents dispersants, les agents de conditionnement et les agents conservateurs.

8. Composition cosmétique selon l'une quelconque des revendications précédentes, **caractérisée en ce qu'**elle comprend au moins un colorant direct additionnel.

9. Composition cosmétique selon l'une quelconque des revendications précédentes, **caractérisée en ce qu'**elle contient au moins un précurseur de colorant d'oxydation choisi parmi les bases d'oxydation et les coupleurs.

10. Composition cosmétique selon la revendication 9, **caractérisée en ce qu'**elle comprend au moins une base d'oxydation choisie parmi les paraphénylènediamines, les bis-phénylalkylènediamines, les paraaminophénols, les orthoaminophénols, les bases hétérocycliques et leurs sels d'addition.

11. Composition cosmétique selon la revendication 10, **caractérisée en ce que** la ou les bases d'oxydation sont présentes en une quantité comprise entre 0,001 à 20 % en poids et de préférence entre 0,005 et 6 % en poids par rapport au poids total de la composition.

12. Composition cosmétique selon l'une des revendications 9 à 11 **caractérisée en ce qu'**elle comprend au moins un coupleur choisi parmi les métaphénylènediamines, les métaaminophénols, les métadiphénols, les coupleurs naphtaléniques, les coupleurs hétérocycliques et leurs sels d'addition.

13. Composition cosmétique selon la revendication 12 **caractérisée en ce que** le coupleur est choisi parmi le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

14. Composition cosmétique selon la revendication 12 ou 13, **caractérisée en ce que** le ou les coupleurs sont présents en une quantité comprise entre 0,001 et 20 % de préférence entre 0,01 et 10 % en poids par rapport au poids total de la composition.

15. Composition cosmétique selon l'une quelconque des revendications précédentes, **caractérisée en ce qu'**elle comprend au moins un solvant hydroxylé tel que l'éthanol, le propylène glycol, le glycérol, les mono éthers de polyols.

16. Composition cosmétique selon l'une des revendications précédentes, **caractérisée en ce qu'**elle comprend au moins un agent oxydant choisi parmi le peroxyde d'hydrogène, le peroxyde d'urée, les bromates de métaux alcalins, les persels, les peracides et les enzymes oxydases, et de préférence le peroxyde d'hydrogène.

17. Procédé de coloration des fibres kératiniques, **caractérisé en qu'**on applique la composition cosmétique telle que définie dans l'une quelconque des revendications 1 à 16 sur les fibres kératiniques, on laisse pauser la composition pendant une période comprise 5 minutes et 1 heure et de préférence entre 15 minutes et 1 heure, puis on rince lesdites fibres.

18. Procédé d'éclaircissement des fibres kératiniques, **caractérisé en qu'**on applique la composition cosmétique telle que définie dans l'une quelconque des revendications 1 à 16 exempte d'agent oxydant sur les fibres kératiniques, on applique simultanément ou séquentiellement à la composition de coloration une composition oxydante, on laisse pauser les compositions pendant une période comprise 5 minutes et 1 heure et de préférence entre 15 minutes et 1 heure, puis on rince, lave au shampooing, rince à nouveau et sèche lesdites fibres.

19. Procédé de coloration des fibres kératiniques, **caractérisé en qu'**on applique la composition cosmétique telle que définie dans l'une quelconque des revendications 1 à 16 comprenant au moins un précurseur de colorant d'oxydation et exempte d'agent oxydant sur les fibres kératiniques, on applique simultanément ou séquentiellement à la composition de coloration une composition oxydante, on laisse pauser les compositions pendant une période comprise 5 minutes et 1 heure et de préférence entre 15 minutes et 1 heure, puis on rince, lave au shampooing, rince à nouveau et sèche lesdites fibres.

20. Dispositif à plusieurs compartiments ou « Kit » de coloration, **caractérisé par le fait qu'**il comporte un premier compartiment renfermant une composition telle que définie selon l'une des revendications 1 à 16 exempte d'agent oxydant et un second compartiment renfermant une composition comprenant un agent oxydant, lorsque l'adjuvant cosmétique contenu dans la composition de coloration est différent d'un agent oxydant.

21. Dispositif selon la revendication 20, **caractérisé par le fait que** le premier compartiment comporte un précurseur de colorant d'oxydation, lorsque l'adjuvant cosmétique contenu dans la composition de coloration est différent d'un précurseur de colorant d'oxydation.

22. Utilisation du composé hydroxyalkylé tel que défini dans l'une quelconque des revendications 1 à 5 pour la coloration des fibres kératiniques, en particulier les fibres kératiniques humaines telles que les cheveux.

23. Utilisation de la composition de coloration telle que définie dans l'une quelconque des revendications 1 à 16 pour la coloration des fibres kératiniques, en particulier les fibres kératiniques humaines telles que les cheveux.
